# EUROPEAN PATENT APPLICATION

(11) **EP 2 693 354 A1**
(43) Date of publication of application: **05.02.2014**
(21) Application number: 13179089.1
(22) Date of filing: 02.08.2013
(51) Int. Cl.: G06F 19/00

(54) **Server, method of controlling server, user terminal apparatus, and method of controlling user terminal apparatus**

(30) Priority: 03.08.2012 KR 20120085352
(71) Applicant: Samsung Electronics Co., Ltd, Gyeonggi-do 443-742 (KR)
(72) Inventor: Min, Chan-hong, Gyeonggi-do (KR); Kim, Bo-min, Gyeonggi-do (KR); Kim, Joon-hwan, Gyeonggi-do (KR)
(74) Representative: Gover, Richard Paul

(57) **Abstract**

Exemplary embodiments may disclose a server including: a communicator configured to communicate with an external apparatus; a storage unit configured to store medicine prescription information about a user; and a controller configured to generate temporary medicine prescription information for providing medicine to the user based on the medicine prescription information if a medicine-taking completion message is not received, and control the communicator to transmit the temporary medicine prescription information to the external apparatus.

## Description

### CROSS-REFERENCE TO RELATED APPLICATION(S)

This application claims priority from Korean Patent Application No. 10-2012-0085352, filed on August 3, 2012, in the Korean Intellectual Property Office, the disclosure of which is incorporated herein by reference in its entirety.

### BACKGROUND

### 1. Field

Exemplary embodiments relate to providing a server, a method of controlling the server, a user terminal apparatus, and a method of controlling the user terminal apparatus. In particular, exemplary embodiments relate to providing a server that provides medicine to a user who runs out of medicine, a method of controlling the server, a user terminal apparatus, and a method of controlling the user terminal apparatus.

### 2. Description of the Related Art

Human longevity has been increased with the development of medicine. Therefore, a number of people who require continuous health care, such as the elderly, chronic patients, adult disease patents, etc., have increased. In particular, a number of people who have chronic diseases, such as high blood pressure or diabetes, have greatly increased.

In general, most people who have chronic diseases take doctor prescribed medicine, instead of being treated at hospitals. In other words, people go to doctors to receive treatments, buy medicine at a pharmacy according to prescriptions written by the doctors, and take the prescribed medicine.

If people who have chronic diseases don't take prescribed medicine, they may not take the prescribed medicine at the prescribed time. Therefore, their diseases may not be improved. In this case, people may go to the doctors again to renew prescriptions and buy the medicine again. However, this process is not convenient for patients.

Therefore, there is a need for a simple method of providing medicine to people who go out without prescribed medicine.

### SUMMARY

Exemplary embodiments may address at least the above problems and/or disadvantages not described above. Also, the exemplary embodiments are not required to overcome the disadvantages described above, and an exemplary embodiment may not overcome any of the problems described above.

The exemplary embodiments may provide a server that effectively provides medicine to a user who runs out of prescribed medicine, a method of controlling the server, a user terminal apparatus, and a method of controlling the user terminal apparatus.

According to an aspect of the exemplary embodiments, there is provided a server including: a communicator configured to communicate with an external apparatus; a storage unit configured to store medicine prescription information about a user; and a controller configured to generate temporary medicine prescription information for providing medicine to the user based on the medicine prescription information if a medicine-taking completion message is not received, and control the communicator to transmit the temporary medicine prescription information to the external apparatus.

The external apparatus may be an electronic apparatus which is installed in a medicine providing place and communicates with the server, and the medicine-taking completion message may be received from a user terminal apparatus.

The external apparatus may be a user terminal apparatus which communicates with the server, and the medicine-taking completion message may be received from the user terminal apparatus.

The medicine prescription information may include at least one of a type of prescribed medicine for the user, a number of times the user takes the prescribed medicine, and a time for taking the prescribed medicine.

The temporary medicine prescription information may be a medicine prescription which is used to prescribe a dose of medicine that is generated based on the medicine prescription information.

The controller may transmit the temporary medicine prescription information to the external apparatus and stores transmission event information in the storage unit if a request for a transmission of the temporary medicine prescription information is received from the external apparatus.

The controller may control not to transmit the temporary medicine prescription information to the external apparatus if the request for the transmission of the temporary medicine prescription information is received after the transmission event is stored.

The external apparatus may be a user terminal apparatus that communicates with the server. If a medicine-taking time in the medicine prescription information passes, the controller may transmit a message to the user terminal apparatus to induce the user to take medicine and, if the medicine-taking completion message is not received at a preset time after the message is transmitted, the controller generates the temporary medicine prescription information.

According to another aspect of the exemplary embodiments, there is provided a user terminal apparatus including: a communicator configured to communicate with a server that stores medicine prescription information about a user; and a controller configured to request the server to transmit temporary medicine prescription information, which is generated based on the medicine prescription information, to provide the user with prescribed medicine according to the medicine prescription information.

The controller may control the communicator to transmit a medicine-taking completion message to the server if the user takes the prescribed medicine according to the medicine prescription information. If the medicine-taking completion message is not received from the user terminal apparatus, the server may generate the temporary medicine prescription information and transmit the temporary medicine prescription information to the user terminal apparatus.

According to another aspect of the exemplary embodiments, there is provided a method of controlling a server. The method may include: generating temporary medicine prescription information for providing medicine to a user based on pre-stored medicine prescription information if a medicine-taking completion message is not received according to the pre-stored medicine prescription information; and transmitting the temporary medicine prescription information to an external apparatus.

The external apparatus may be an electronic apparatus which is installed in a medicine providing place to communicate with the server, and the medicine-taking completion message may be received from a user terminal apparatus.

The external apparatus may be a user terminal apparatus which communicates with the server, and the medicine-taking completion message may be received from the user terminal apparatus.

The medicine prescription information may include at least one of a type of prescribed medicine for the user, a number of times the user takes the prescribed medicine, and a time for taking the prescribed medicine.

The temporary medicine prescription information may be a medicine prescription which is used to prescribe a dose of medicine generated based on the medicine prescription information.

The method may further include: transmitting the temporary medicine prescription information to the external apparatus and storing transmission event information if a request for a transmission of the temporary medicine prescription information is received from the external apparatus.

The temporary medicine prescription information may not be transmitted to the external apparatus if the request for the transmission of the temporary medicine prescription information is received after the transmission event is stored.

The external apparatus may be a user terminal apparatus that communicates with the server. The method may further include: transmitting a message to the user terminal to induce the user to take medicine; and generating the temporary medicine prescription information if the medicine-taking completion message is not received for a preset time after the message is transmitted.

According to another aspect of the exemplary embodiments, there is provided a method of controlling a user terminal apparatus configured to communicate with a server that stores medicine prescription information about a user. The method may include: requesting the server to transmit temporary medicine prescription information generated based on the medicine prescription information to provide the user with prescribed medicine according to the medicine prescription information; and receiving the temporary medicine prescription information from the server.

The method may further include: transmitting a medicine-taking completion message to the server if the user takes the prescribed medicine according to the medicine prescription information. The server may generate the temporary medicine prescription information and transmit the temporary medicine prescription information to the user terminal apparatus if the medicine-taking completion message is not received from the user terminal apparatus.

According to another aspect of the exemplary embodiments, there is provided a method of a portable apparatus for a prescribed medicine. The method may include: receiving medicine prescription information; storing the medicine prescription information; and notifying a user that a medicine-taking time has arrived based on the medicine prescription information.

### BRIEF DESCRIPTION OF THE DRAWINGS

The above and/or other aspects will be more apparent by describing certain exemplary embodiments with reference to the accompanying drawings, in which:

FIG. 1 is a view illustrating a medicine-taking management system according to an exemplary embodiment;

FIG. 2 is a timing diagram illustrating operations of the medicine-taking management system of FIG. 1, according to an exemplary embodiment;

FIG. 3 is a timing diagram illustrating operations of the medicine-taking management system of FIG. 1, according to another exemplary embodiment;

FIG. 4 is a block diagram illustrating a structure of a server according to an exemplary embodiment;

FIG. 5 is a block diagram illustrating a structure of a user terminal apparatus according to an exemplary embodiment;

FIG. 6 is a block diagram illustrating a detailed structure of the user terminal apparatus of FIG. 5;

FIG. 7 is a view illustrating a medicine-taking management system according to another exemplary embodiment;

FIG. 8 is a block diagram illustrating a structure of a portable apparatus according to an exemplary embodiment;

FIG. 9 is a flowchart illustrating a method of controlling a server according to an exemplary embodiment; and

FIG. 10 is a flowchart illustrating a method of controlling a user terminal apparatus according to an exemplary embodiment.

### DETAILED DESCRIPTION OF THE EXEMPLARY EMBODIMENTS

Exemplary embodiments are described in greater detail with reference to the accompanying drawings.

In the following description, the same drawing reference numerals are used for the same elements, even in different drawings. The matters defined in the description, such as detailed construction and elements, are provided to assist in a comprehensive understanding of the exemplary embodiments. Thus, it is apparent that the exemplary embodiments can be carried out without those specifically defined matters. Also, well-known functions or constructions are not described in detail since they would obscure the exemplary embodiments with unnecessary detail.

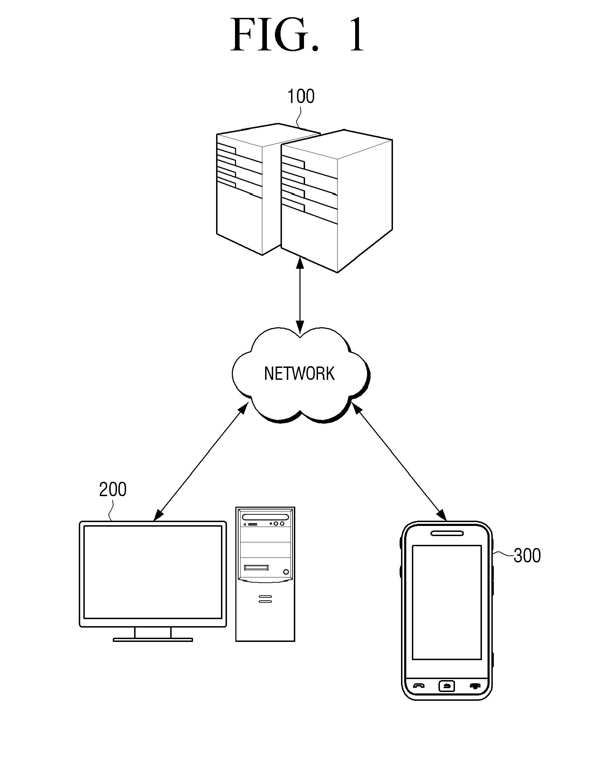

FIG. 1 is a view illustrating a medicine-taking management system according to an exemplary embodiment. Referring to FIG. 1, the medicine-taking management system, according to the present exemplary embodiment, includes a server 100, an electronic apparatus 200, and a user terminal apparatus 300. Here, the server 100, the electronic apparatus 200, and the user terminal apparatus 300 may be connected to one another through a network, such as the Internet, etc.

The electronic apparatus 200 may be realized as a desktop personal computer (PC) as shown in FIG. 1, but this is only an example. Therefore, the electronic apparatus 200 may be realized as various types of electronic apparatuses, such as a portable phone, a smart phone, a tablet PC, etc. The user terminal apparatus 300 may be a portable phone, such as a smart phone as shown in FIG. 1, but this is only an example. Therefore, the user terminal apparatus 300 may be realized as various types of electronic apparatuses, such as a tablet PC, a personal digital assistant (PDA), a navigation system, etc.

. The server 100 stores medicine prescription information about a user. The medicine prescription information may include at least one of a type of prescribed medicine, a method of taking the prescribed medicine, a time for taking the prescribed medicine, and information about the number of times the prescribed medicine has been taken.

The server 100 may generate temporary medicine prescription information based on the medicine prescription information and transmit the temporary medicine prescription information to the electronic apparatus 200 or the user terminal apparatus 300. The medicine prescription information is used to prescribe a dose of medicine.

The electronic apparatus 200 is installed in a medicine providing place such as a pharmacy, etc., to receive the temporary medicine prescription information from the server 100. For example, the electronic apparatus 200 may access the server 100 through the Internet and receive temporary medicine prescription information generated for each user.

The user terminal apparatus 300 may receive the temporary medicine prescription information from the server 100 and store the temporary medicine prescription information.

The user terminal apparatus 300 may request the server 100 to transmit the temporary medicine prescription information and receive the temporary medicine prescription information from the server 100.

If the server 100 does not receive a taking completion message from the user terminal apparatus 300 for a preset time, the server 100 may transmit the temporary medicine prescription information to the user terminal apparatus 300. The taking completion message refers to a message indicating the user has taken the prescribed medicine

As described above, the medicine-taking management system, according to the present exemplary embodiment, generates the temporary medicine prescription information. The medicine-taking management system may induce the user to easily buy medicine, when the user runs out of the prescribed medicine. In particular, the temporary medicine prescription information prescribes a dose of medicine. Thus, the temporary medicine prescription information may be used to prevent the user from unnecessarily buying a large amount of medicine and abusing the medicine.

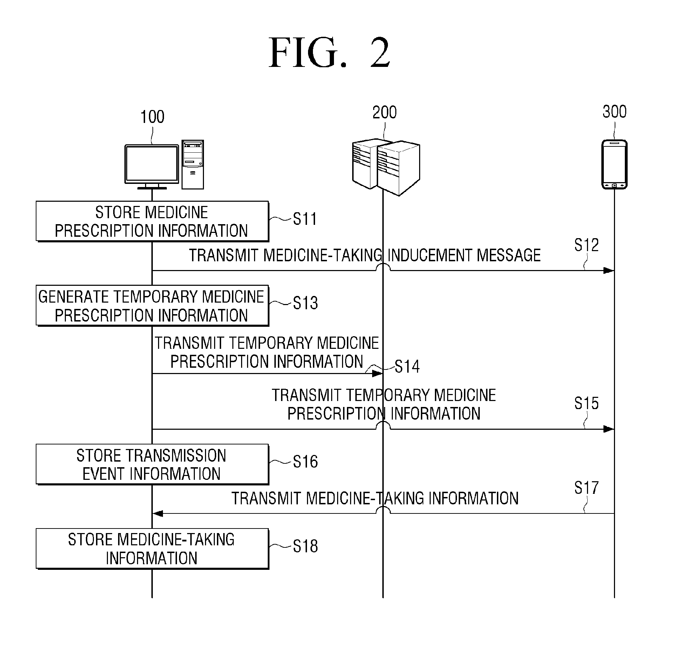

FIG. 2 is a timing diagram illustrating operations of the medicine-taking management system of FIG. 1, according to an exemplary embodiment.

Referring to FIG. 2, the server 100 stores medicine prescription information in operation S11. If a doctor or a nurse accesses the server 100 through the Internet, etc., personal information of a user is input (e.g., an ID, a password, and an ID card number of the user), and the medicine prescription information is input, the server 100 may store medicine prescription information of each user input by the doctor or the nurse.

The medicine prescription information may include at least one of a type of medicine prescribed for the user, the number of times the user takes the prescribed medicine, a method of taking the prescribed medicine, and a time for taking the prescribed medicine. In other words, the server 100 may store a type of medicine that the user is taking according to a prescription of a doctor, a method of taking the medicine, a date for taking the prescribed medicine according to a treatment date, a taking time determined according to a meal time of the user, etc.

In operation S12, the server 100 transmits a medicine-taking inducement message to the user terminal apparatus 300. If the server 100 does not receive the medicine-taking completion message from the user terminal apparatus 300 even after a time when the user is to take the medicine according to the medicine prescription information, the server 100 may transmit the medicine-taking inducement message to the user terminal apparatus 300.

The medicine-taking inducement message is a guide message for inducing the user to take the medicine and may include information about the medicine that the user is to take (e.g., a type of the medicine, a method of taking the medicine, etc.), information about a time for taking the medicine, etc. In detail, the server 100 may transmit the medicine-taking inducement message in a Short Message Service (SMS) or Automatic Response Service (ARS) form to the user terminal apparatus 300. The server 100 may match information (e.g., a phone number) about a user terminal apparatus with each user and store the matched information.

In operation S13, the server 100 generates temporary medicine prescription information. If the sever 100 does not receive the medicine-taking completion message from the user terminal apparatus 300 even after the server 100 transmits the medicine-taking inducement message to the user terminal apparatus 300 and then a preset time passes, the server 100 may generate the temporary medicine prescription information. The temporary medicine prescription information may be used to prescribe a dose of medicine which is generated based on medicine prescription information.

In other words, if the server 100 does not receive the medicine-taking completion message from the user terminal apparatus 300 for a preset time, the server 100 determines that the user does not have the medicine and generates a temporary medicine prescription so that the user may buy the medicine again. The server 100 generates a medicine prescription for providing a dose of medicine to the user, based on a type of medicine, a method of taking the medicine, etc., which are included in the medicine prescription information. This prevents the user from unnecessarily buying a large amount of medicine and abusing the medicine.

In operation S14, the server 100 transmits the temporary medicine prescription information to the electronic apparatus 200. If a pharmacist accesses the server 100 through the electronic apparatus 200 installed in a medicine providing place, such as a pharmacy, etc., and inputs personal information of the user on a webpage provided by the server 100 to request a transmission of the temporary medicine prescription information, the server 100 may transmit the temporary medicine prescription information to the electronic apparatus 200. Therefore, the user may buy a dose of medicine that has been prescribed to the user at the medicine providing place.

In operation S15, the server 100 transmits the temporary medicine prescription information to the user terminal apparatus 300. For example, the server 100 may transmit the temporary medicine prescription information in an SMS form to the user terminal apparatus 300. Therefore, the user may go to the medicine providing place with the user terminal apparatus 300 to buy a dose of the medicine that has been prescribed to the user.

The server 100 transmits the temporary medicine prescription information to both the electronic apparatus 200 and the user terminal apparatus 300 in FIG. 2, but this is described for convenience. In other words, the server 100 may transmit the temporary medicine prescription information to the electronic apparatus 200 or the user terminal apparatus 300. Further, the server 100 may transmit the temporary medicine prescription information to both the electronic apparatus 200 and the user terminal apparatus 300.

In operation S16, the server 100 stores transmission event information. If the server 100 transmits the temporary medicine prescription information to the electronic apparatus 200 or the user terminal apparatus 300, the server 100 stores information indicating that the temporary medicine prescription information has been completely transmitted. Therefore, if the electronic apparatus 200 or the user terminal apparatus 300 re-requests a transmission of the temporary medicine prescription information that has been completely transmitted, the server 100 may not transmit the temporary medicine prescription information.

In operation S17, the user terminal apparatus 300 transmits medicine-taking information to the server 100. In operation S18, the server 100 stores the medicine-taking information.

The user may access the server 100 through the user terminal apparatus 300 and write information indicating that the user has taken the medicine provided according to the temporary medicine prescription information, on a webpage provided by the server 100 and transmit the medicine-taking information to the server 100. Therefore, a doctor or a nurse may recognize that additional medicine has been provided to the user, and write an appropriate prescription to the user in consideration of a medicine-taken state of the user.

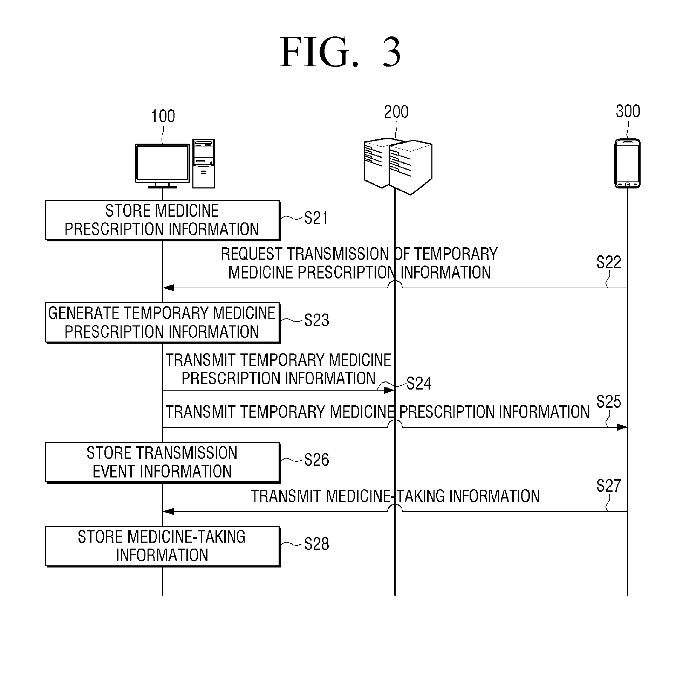

FIG. 3 is a timing diagram illustrating operations of the medicine-taking management system of FIG. 1, according to another exemplary embodimen. The present exemplary embodiment of FIG. 3 is different from the previous exemplary embodiment of FIG. 2 only in that the user terminal apparatus 300 additionally requests a transmission of temporary medicine prescription information, and the server 100 generates the temporary medicine prescription information according to the request for the transmission of the temporary medicine prescription information and transmits the temporary medicine prescription information to the electronic apparatus 200 or the user terminal apparatus 300.

Therefore, operation S21 of FIG. 3 is equal to operation S11 of FIG. 2, and operations S23 through 28 of FIG. 3 are equal to operations S13 through 18 of FIG. 2. Thus, repeated descriptions are omitted.

Referring to FIG. 3, the user terminal apparatus 300 requests the server 100 to transmit the temporary medicine prescription information.

A user may access the server 100 through the user terminal apparatus 300 and request the server 100 on a webpage to transmit the temporary medicine prescription information. In other words, if the user runs out of medicine, and then a medicine-taking time arrives, the user may request the server 100, through the user terminal apparatus 300, to transmit the temporary medicine prescription information and buy medicine based on temporary medicine prescription information generated according to the request.

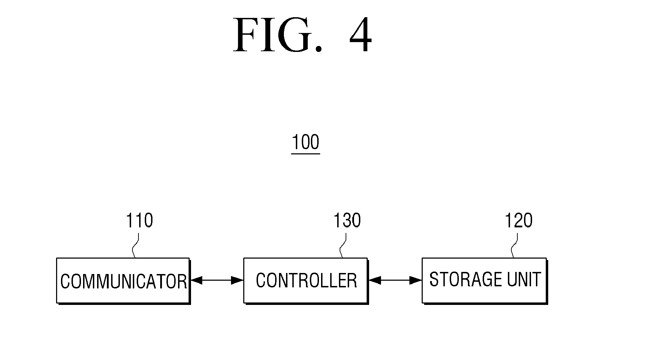

FIG. 4 is a block diagram illustrating a structure of the server 100, according to an exemplary embodiment. Referring to FIG. 4, the server 100 includes a communicator 110, a storage unit 120, and a controller 130.

The communicator 110 communicates with an external apparatus. The external apparatus may be the electronic apparatus 200 which is installed in a medicine providing place such as a pharmacy, or the user terminal apparatus 300 which is carried by a user.

For example, the communicator 110 may be realized as a network interface card (not shown) to communicate with the electronic apparatus 200 and the user terminal apparatus 300 through the Internet, etc. The communicator 110 may also access a mobile communication network, according to various types of mobile communication standards, to transmit an SMS or ARS message to the user terminal apparatus 300.

The storage unit 120 stores medicine prescription information about a user. If a doctor or a nurse accesses the server 100, inputs personal information of the user, and inputs medicine prescription information, the storage unit 120 may match medicine prescription information with each user and store the matched medicine prescription information.

The medicine prescription information may include at least one of a type of medicine prescribed for the user, the number of times the user takes the prescribed medicine, a method of taking the prescribed medicine, and a time for taking the prescribed medicine. For example, the storage unit 120 may store a type of medicine that the user is taking according to a prescription of the doctor, a method of taking the prescribed medicine, a date for taking the prescribed medicine, wherein the date is determined according to a treatment date, a taking time determined according to a meal time of the user, etc.

The storage unit 120 may also store a network address of the electronic apparatus 200. For example, if the server 100 communicates with the electronic apparatus 200 through the Internet, the storage unit 120 may store an Internet Protocol (IP) address of the electronic apparatus 200 installed in medicine providing places of various areas.

The storage unit 120 may store information about the user. In detail, the storage unit 120 may match information (e.g., a phone number) about the user terminal apparatus 300 with each user and store the matched information.

As described above, the information about the user terminal apparatus 300 is stored in order to transmit a medicine-taking inducement message to the user. Therefore, the storage unit 120 may also store information about the user terminal apparatus 300 of the user as a patient, a caretaker, a guardian, etc. The information about the user terminal apparatus 300 may be input together with the medicine prescription information, and may be stored in the storage unit 120.

The controller 130 controls an overall operation of the server 100. In particular, if a medicine-taking completion message is not received according to the medicine prescription information, the controller 130 may generate temporary medicine prescription information for providing the medicine to the user based on the medicine prescription information and control the communicator 110 to transmit the temporary medicine prescription information to the external apparatus.

The external apparatus may be the electronic apparatus 200 which is installed in a medicine providing place to communicate with the server 100 or the user terminal apparatus 300 which communicates with the server 100.

The medicine-taking completion message notifies the server 100 that the user has taken the medicine according to a prescription, and may be received from the user terminal apparatus 300. The user may access the server 100 through the user terminal apparatus 300, and input personal information of the user on a webpage provided by the server 100 to log on the server 100. The user may input a type of medicine that the user has taken, a time when the user has taken the medicine, etc., to notify the server 100 that the user has taken the medicine according to the prescription.

The controller 130 may transmit a message for inducing medicine taking to the user terminal apparatus 300, after a medicine taking time included in the medicine prescription information passes, and if a medicine-taking completion message is not received for a preset time after transmitting the message, generate temporary medicine prescription information. In other words, if the medicine taking time passes when the medicine-taking completion message is not received, the controller 130 may transmit the message for inducing the medicine-taking to the user terminal apparatus 300.

The medicine-taking inducement message is to induce the user to take the medicine and may include a message indicating that a time for taking the medicine has passed and information about a type of medicine prescribed for the user, a method of taking the prescribed medicine, and a time for taking the prescribed medicine. Also, the medicine-taking inducement message may be transmitted to the user terminal apparatus 300 in an SMS or ARS form during every preset time interval.

In other words, if the medicine-taking completion message is not received for a preset time after the medicine-taking inducement message is transmitted, the controller 130 may determine that the user does not have the medicine and generate temporary medicine prescription information so that the user is temporarily provided with the medicine in a medicine providing place.

The temporary medicine prescription information may be a medicine prescription to prescribe a dose of medicine based on medicine prescription information. The controller 130 may generate a medicine prescription to provide a dose of medicine to the user, based on the type of medicine, the method of taking the medicine, etc., which are included in the medicine prescription information.

If a medicine-taking time does not pass, and the user terminal apparatus 300 requests a transmission of the temporary medicine prescription information, the controller 130 may generate the temporary medicine prescription information. In other words, if the user runs out of medicine and recognizes that the medicine-taking time arrives, the user may request the server 100, through the user terminal apparatus 300, to transmit the temporary medicine prescription information.

For example, the user may access the server 100 through the user terminal apparatus 300 and input personal information on a webpage, provided by the server 100, to log in the server 100. The user may select an object (e.g., an icon) provided on a webpage screen to request the server 100 to transmit the temporary medicine prescription information. If the request for the transmission of the temporary medicine prescription information is received from the user terminal apparatus 300, the controller 130 may generate the temporary medicine prescription information according to the request of the user terminal apparatus 300.

The controller 130 may store the generated temporary medicine prescription information in the storage unit 120. The controller 130 may match temporary medicine prescription information with each user and store the matched temporary medicine prescription information.

If a request for a transmission of the temporary medicine prescription information is received from the external apparatus, the controller 130 may transmit the temporary medicine prescription information to the external apparatus and store transmission event information in the storage unit 120.

If a pharmacist accesses the server 100 through the electronic apparatus 200 installed in the medicine providing place and inputs personal information of the user to request temporary medicine prescription information, the controller 130 may transmit temporary medicine prescription information matching the personal information of the user to the electronic apparatus 200.

In this case, if the controller 130 compares an IP address stored in the storage unit 120 with an IP address of the electronic apparatus 200 that has accessed the server 100, and the IP address of the electronic apparatus 200 is pre-stored in the storage unit 120, the controller 130 may transmit temporary medicine prescription information to the electronic apparatus 200 through the Internet. Therefore, temporary medicine prescription information may be provided only to the electronic apparatus 200 which is installed in the medicine providing place. The medicine providing place may have a person, i.e., a pharmacist, which has a right to operate the electronic apparatus 200.

If the user inputs personal information through the user terminal apparatus 300 to log in the server 100 and requests temporary medicine prescription information, the controller 130 may transmit temporary medicine prescription information matching the personal information of the user to the user terminal apparatus 300. In this case, the controller 130 may transmit the temporary medicine prescription information in an SMS form to the user terminal apparatus 300 through a mobile communication network.

If the temporary medicine prescription information is transmitted to the external apparatus, the controller 130 may store transmission event information indicating that the temporary medicine prescription information has been completely transmitted, in the storage unit 120. If the transmission event information is stored, and the request for the transmission of the temporary medicine prescription information is received, the controller 130 may control not to transmit the temporary medicine prescription information to the external apparatus.

The controller 130 may store information, about whether temporary medicine prescription information about each user has been completely transmitted, in the storage unit 120 to determine whether temporary medicine prescription information has been provided to a user who has requested a transmission of temporary medicine prescription information. Therefore, if a user who has already been provided with temporary medicine prescription information requests a transmission of the temporary medicine prescription information, the controller 130 may control the communicator 110 not to transmit the temporary medicine prescription information to the electronic apparatus 200 and the user terminal apparatus 300. As a result, a user is prevented from being provided with medicine a plurality of times according to temporary medicine prescription information. Therefore, a large amount of medicine is prevented from being unnecessarily provided to the user.

If medicine-taking information is received from the user terminal apparatus 300, the controller 130 may store the medicine-taking information in the storage unit 120.

In other words, if the user is provided with a dose of medicine and takes the medicine according to temporary medicine prescription information, the user may input personal information of the user through the user terminal apparatus 300 to log in the server 100 and write information indicating that the user has taken the medicine, on a webpage provided by the server 100 to transmit the medicine-taking information to the server 100. If the medicine-taking information is received from the user terminal apparatus 300, the controller 130 may store match received medicine-taking information with each user and store the matched medicine-taking information in the storage unit 120. Therefore, the user may let the doctor or the nurse know that the user has taken the medicine according to the temporary medicine prescription information.

The controller 130 may control the communicator 110 to transmit a message for inducing the user to have medicine to the user terminal apparatus 300 based on position information of the user received from the user terminal apparatus 300. For this purpose, the storage unit 120 may match Global Positioning System (GPS) information about a residence of each user with each user and store the matched GPS information.

The controller 130 compares the GPS information received from the user terminal apparatus 300 with pre-stored GPS information to determine whether the user has left the residence. Therefore, if it is determined that the user has left the residence, the controller 130 may transmit a message for inquiring whether the user has the medicine, to the user terminal apparatus 300. The controller 130 may transmit information about a type of medicine that the user is to take, a method of taking the medicine, and a time for taking the medicine.

In the above-described exemplary embodiment, the server 100 transmits temporary medicine prescription information to the electronic apparatus 200 or the user terminal apparatus 300 according to a request of the electronic apparatus 200 or the user terminal apparatus 300. The server 100 may transmit temporary medicine prescription information with respect to only a request satisfying a preset condition.

In other words, only if the electronic apparatus 200 accesses the server 100 within a preset time to request a transmission of the temporary medicine prescription information or accesses the server 100 in a particular place to request a transmission of the temporary medicine prescription information, the controller 130 may control to transmit the temporary medicine prescription information.

For example, only if the electronic apparatus 200 accesses the server 100 from 1 PM to 2 PM to request a transmission of the temporary medicine prescription information, the controller 130 may transmit the temporary medicine prescription information. Alternatively, only if the electronic apparatus 200 having a particular IP address accesses the server 100 to request a transmission of the temporary medicine prescription information, the controller 130 may transmit the temporary medicine prescription information.

Also, in the above-described exemplary embodiment, the temporary medicine prescription information is a prescription which provides a dose of medicine to a user. However, this is only an example. Therefore, the temporary medicine prescription information may include a prescription which provides a larger amount than a prescribed dose of medicine.

In other words, when the user requests a transmission of the temporary medicine prescription information through the user terminal apparatus 300, the user may select doses of medicine that the user wants to be prescribed, on a webpage provided by the server 100. For example, a user may select a prescription of a larger amount than a prescribed dose of medicine.

Therefore, the controller 130 may control to generate a prescription for providing a larger amount than a prescribed dose of medicine based on doses of medicine selected by the user, and transmit the prescription to the electronic apparatus 200 or the user terminal apparatus 300.

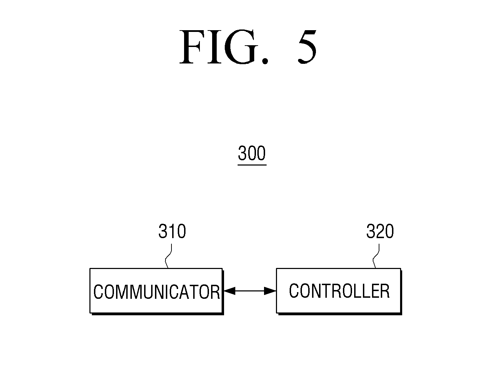

FIG. 5 is a block diagram illustrating a structure of the user terminal apparatus 300, according to an exemplary embodiment. Referring to FIG. 5, the user terminal apparatus 300 includes a communicator 310 and a controller 320.

The communicator 310 communicates with the server 100 which stores medicine prescription information about a user. For this purpose, the communicator 310 may include wireless communication module (not shown) to access the server 100 through the Internet.

The wireless communication module is connected to an external network to perform communications according to a wireless communication protocol, such as WiFi, IEEE, etc. The wireless communication module may further include a mobile communication module that accesses a mobile communication network to perform communications according to various types of mobile communication standards, such as 3rD Generation (3G), 3rd Generation Partnership Project (3GPP), Long Term Evolution (LTE), etc.

The communicator 310 may receive an SMS or ARS message from the server 100 through the mobile communication network.

The controller 320 controls an overall operation of the user terminal apparatus 300. In particular, the controller 320 may request the server 100 to transmit temporary medicine prescription information, which is generated based on the medicine prescription information, to provide the user with medicine prescribed according to the medicine prescription information.

The controller 320 may drive an application installed in the user terminal apparatus 300 to access the server 100 and request the server 100 to transmit the temporary medicine prescription information based on a user command.

For example, the user may input personal information of the user onto a webpage provided by the server 100 to log on to the server 100 and select an object (e.g., an icon) for requesting a transmission of the temporary medicine prescription information provided on the corresponding webpage to request the server 100 to transmit the temporary medicine prescription information.

Therefore, the server 100 may generate the temporary medicine prescription information based on the medicine prescription information and transmit the generated temporary medicine prescription information to the electronic apparatus 200 or the user terminal apparatus 300.

If the user takes the medicine prescribed according to the medicine prescription information, the controller 320 may control the communicator 310 to transmit a medicine-taking completion message to the server 100.

The controller 320 may control to display a webpage screen provided by the server 100 on a screen provided in the user terminal apparatus 300, receive medicine-taking completion message from the user, and transmit the medicine-taking completion message to the server 100. For example, the user may input personal information of the user on a webpage provided by the server 100 to log on to the server 100 and input a type of medicine that the user has taken, a time for taking the medicine, etc., onto the corresponding webpage to notify the server 100 that the user has taken the medicine.

If the medicine-taking completion message is not received from the user terminal apparatus 300, the server 100 may generate temporary medicine prescription information and transmit the temporary medicine prescription information to the user terminal apparatus 300. This has been described above with reference to FIGS. 1 through 4.

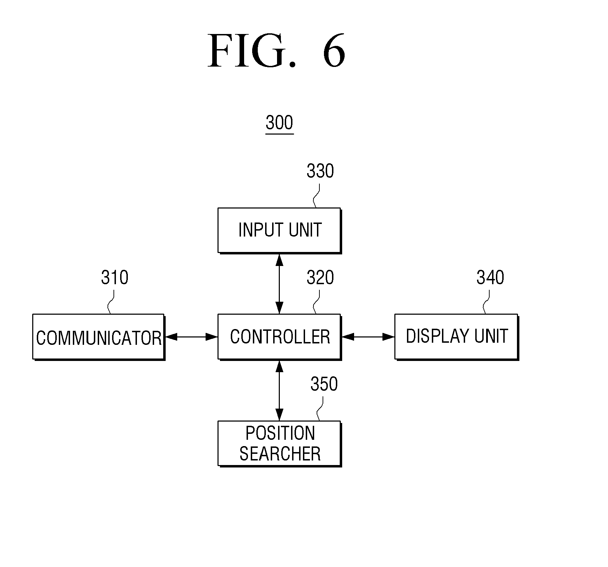

FIG. 6 is a block diagram illustrating a detailed structure of the user terminal apparatus 300 of FIG. 5, according to an exemplary embodiment. Referring to FIG. 6, the user terminal apparatus 300 further includes an input unit 330, a display unit 340, and a position searcher 350 besides the elements of FIG. 5. The same elements of FIG. 6 as those of FIG. 5 have the same functions. Thus, their detailed descriptions are omitted.

The input unit 330 receives various types of user controls and transmits the various types of user controls to the controller 320 and may be realized as an input panel. The input panel may be a touch pad type, a keypad type including various types of functional keys, numerical keys, special keys, character keys, etc., or a touch screen type.

In particular, the input unit 330 may be realized as a touch screen type, which forms an interactive layer structure with a display panel, to display various types of screens and receive various types of user controls for controlling a function of the user terminal apparatus 300.

The input unit 330 may select various types of objects (e.g., icons) displayed on a webpage provided by the server 100 or may receive a user control for inputting particular information on a webpage. For example, if an information input window displayed on the webpage is selected, the input unit 330 may display a virtual keyboard to receive personal information of the user and information about whether the user has taken medicine.

The display unit 340 may provide various types of display screens which may be provided through the user terminal apparatus 300. In particular, the display unit 340 may display a webpage screen provided by the server 100. The display unit 340 may be realized as a touch screen type which forms an interactive layer structure with a touch pad to detect a position, an area, and a pressure, etc., of a touch input.

The position searcher 350 includes a GPS module to search a position using a GPS signal. The GPS module receives signals from a plurality of GPS satellites to calculate a distance between the GPS satellites and the user terminal apparatus 300 using a time difference between a transmission time and a reception time. The position searcher 350 may calculate a current position of the user terminal apparatus 300 using an operation method, such as a trilateration synthetically in consideration of distances calculated between the plurality of GPS satellites and the user terminal apparatus 300, and positions of the GPS satellites, etc.

The controller 320 may transmit GPS information of the user terminal apparatus 300 searched by the position searcher 350 to the server 100. The controller 320 may control the communicator 310 to transmit the searched GPS information according to a preset time interval or a request of the server 100.

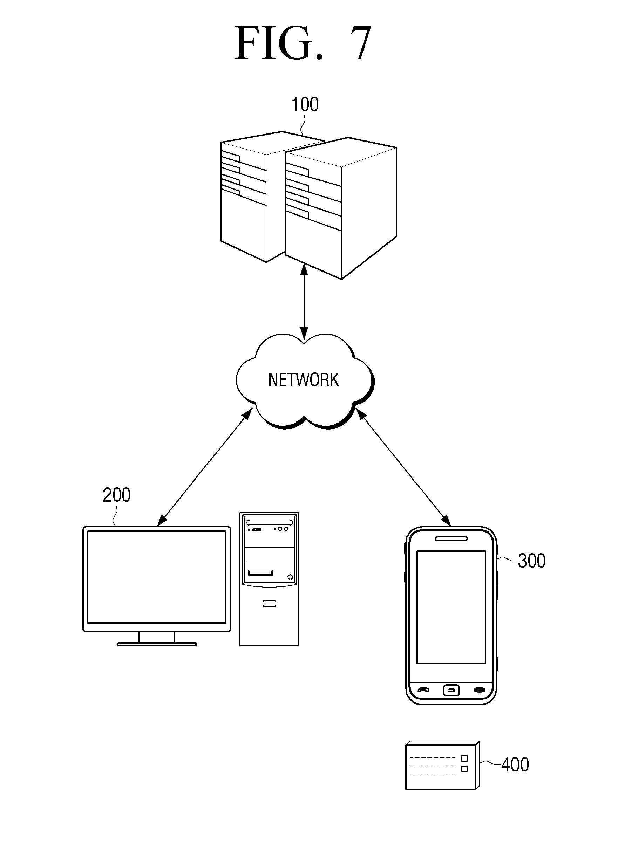

FIG. 7 is a view illustrating a medicine-taming management system according to another exemplary embodiment. Referring to FIG. 7, the medicine-taking management system includes a server 100, an electronic apparatus 200, a user terminal apparatus 300, and a portable apparatus 400. The medicine-taking management system of FIG. 7 is different from the medicine-taking management system of FIG. 1 in that the medicine-taking management system includes the portable apparatus 400. Thus, repeated detailed description is omitted.

The portable apparatus 400 receives and stores the medicine prescription information and notifies the user that a medicine-taking time of the user arrives through various methods.

The portable apparatus 400 may determine whether a medicine-taking time of the user has arrived, based on the medicine-taking time included in the medicine prescription information, and output a notification sound, light, or generate vibrations if the medicine-taking time arrives. The portable apparatus 400 may be attached to a medicine container distributed to the user.

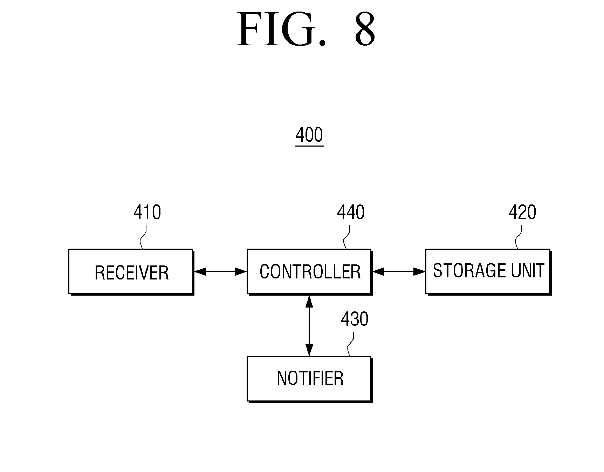

FIG. 8 is a block diagram illustrating a structure of the portable apparatus 400, according to an exemplary embodiment. Referring to FIG. 8, the portable apparatus 400 includes a receiver 410, a storage unit 420, a notifier 430, and a controller 440.

The receiver 410 receives medicine prescription information. The receiver 410 may access the server 100 through the Internet to receive the medicine prescription information from the server 100. Also, the receiver 410 may be a near field communication (NFC) tag which receives the medicine prescription information through tagging with an NFC reader which stores the medicine prescription information.

The storage unit 420 stores the medicine prescription information. The storage unit 420 may match medicine prescription information with each user, and store the matched medicine prescription information.

The notifier 430 may be various types to notify the user that the medicine-taking time has arrived. For example, the notifier 430 may include a vibration module (not shown) to generate constant vibrations or may generate light through a light-emitting device (not shown), such as a light-emitting diode (LED). Also, the notifier 430 may be a speaker to output a notification sound if the medicine-taking time arrives.

The controller 440 controls an overall operation of the portable apparatus 400. In particular, the controller 440 may determine whether the medicine-taking time of the user has arrived, based on the medicine prescription information and control the notifier 430 to notify the user whether the medicine-taking time has arrived.

The controller 440 may determine whether the medicine-taking time included in the medicine prescription information has arrived, and generate vibrations or light through the notifier 430 or output a preset notification sound if the medicine-taking time arrives. In this case, the controller 440 may control the notifier 430 to notify the user every preset time interval whether the medicine-taking time has arrived.

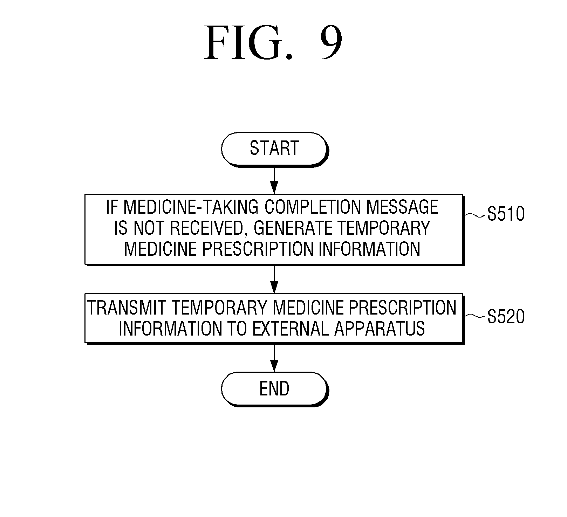

FIG. 9 is a flowchart illustrating a method of controlling a server according to an exemplary embodiment.

If a medicine-taking completion message is not received according to pre-stored medicine prescription information, the server generates temporary medicine-taking information for providing medicine to a user, based on the medicine prescription information in operation S510.

If a medicine-taking time included in the medicine prescription information passes, the server transmits a message for inducing the user to take medicine to a user terminal apparatus. If a medicine-taking completion message is not received for a preset time after the message is transmitted, the server may generate temporary medicine-taking information.

The medicine prescription information may include at least one of a type of medicine prescribed for the user, the number of times the user takes the prescribed medicine, and a time for taking the prescribed medicine. Also, the temporary medicine prescription information may be a medicine prescription to prescribe a dose of medicine generated based on the medicine prescription information.

In other words, the server generates the medicine prescription to provide a dose of medicine to the user, based on the type of medicine, a medicine-taking method, etc., included in the medicine prescription information, in order to prevent the user from unnecessarily buying a large amount of medicine and abusing medicine.

The medicine-taking completion message notifies the server that the user has taken medicine according to the medicine prescription and may be received from the user terminal apparatus.

In operation S520, the server transmits the temporary medicine prescription information to an external apparatus. The external apparatus may be an electronic apparatus that is installed in a medicine providing place to communicate with the server or a user terminal apparatus that communicates with the server. The medicine-taking completion message may be received from the user terminal apparatus. Therefore, the user may visit the medicine providing place or visit the medicine providing place with the user terminal apparatus to buy a dose of medicine which has been prescribed for the user.

According to an exemplary embodiment, if a request for a transmission of temporary medicine prescription information is received from the external apparatus, the server may transmit the temporary medicine prescription information to the external apparatus and store transmission event information. If the transmission event information is stored, and then the request for the transmission of the temporary medicine prescription information is received, the server does not transmit the temporary medicine prescription information to the external apparatus. Therefore, a user who has already been provided with the medicine according to temporary medicine prescription information is prevented from being provided with additional medicine.

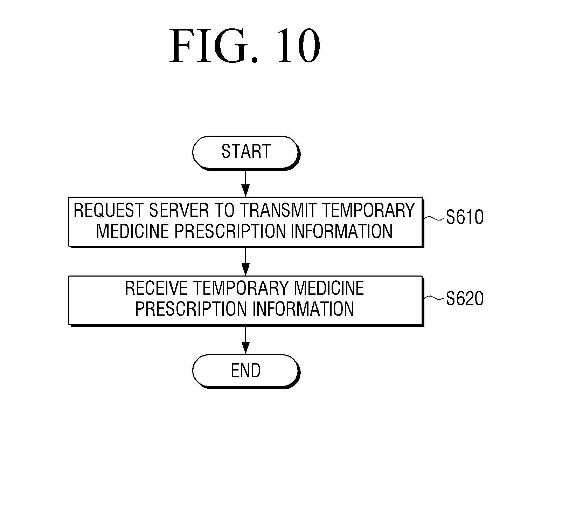

FIG. 10 is a flowchart illustrating a method of controlling a user terminal apparatus according to an exemplary embodiment. In particular, FIG. 10 illustrates the method of controlling the user terminal apparatus which communicates with a server that stores medicine prescription information about a user.

In operation S610, the user terminal apparatus requests the server to transmit temporary medicine prescription information generated based on medicine prescription information, in order to provide the user with medicine prescribed according to the medicine prescription information. The user may access the server through the user terminal apparatus and input personal information of the user on a webpage provided by the server to log in the server. The user may also select an object for requesting the transmission of the temporary medicine prescription information on the webpage to request the server to transmit the temporary medicine prescription information.

In operation S620, the user terminal apparatus receives the temporary medicine prescription information from the server. In this case, the temporary medicine prescription information may be received in an SMS form. The temporary medicine prescription information includes a medicine prescription which provides a dose of medicine to the user based on a type of medicine, a method of taking the medicine, etc., included in the medicine prescription information. Therefore, the user may visit a pharmacy with the user terminal apparatus to buy a dose of medicine according to the medicine prescription. Although the user runs out of the medicine, the user may easily buy medicine.

According to an exemplary embodiment, if the user takes medicine prescribed according to medicine prescription information, the user terminal apparatus may transmit a medicine-taking completion message to the server. In this case, if the server does not receive the medicine-taking completion message from the user terminal apparatus, the server may generate temporary medicine prescription information and transmit the temporary medicine prescription information to the user terminal apparatus.

The medicine-taking completion message notifies the server that the user has taken medicine according to a prescription and may input a type of medicine that the user has taken, a taking time, etc., onto a webpage to notify the server that the user has taken the medicine according to the prescription.

If the medicine-taking completion message is not received from the user terminal apparatus, the server may determine that the user does not have medicine to generate temporary medicine prescription information and transmit the generated temporary medicine prescription information to the user terminal apparatus.

According to various exemplary embodiments, medicine may be provided to a user who runs out of medicine. Therefore, user convenience may be improved, and the user may effectively manage their health.

Exemplary embodiments may provide a non-transitory computer-readable medium which stores a program that sequentially performs a method of controlling a server and a method of controlling a user terminal apparatus.

The non-transitory computer-readable medium refers to a medium which does not store data for a short time such as a register, a cache memory, a memory, or the like but semi-permanently stores data, and is readable by a device. The above-described applications or programs may be stored and provided on a non-transitory computer readable medium, such as a CD, a DVD, a hard disk, a blue-ray disk, a universal serial bus (USB), a memory card, a ROM, etc.

A bus has not been illustrated in the above-described block diagrams illustrating the server and the user terminal apparatus. However, communications between elements of the server and elements of the user terminal apparatus may be performed through the bus. Also, each device may further include a processor, such as a central processing unit (CPU), a microprocessor, etc., that performs the above-described operations.

The foregoing exemplary embodiments and advantages are merely exemplary and are not to be construed as limiting. The present teaching can be readily applied to other types of apparatuses. Also, the description of the exemplary embodiments is intended to be illustrative, and not to limit the scope of the claims. Therefore, many alternatives, modifications, and variations will be apparent to those skilled in the art.

## Claims

1. A server comprising:
a communicator configured to communicate with an external apparatus;
a storage unit configured to store medicine prescription information about a user; and
a controller configured to generate temporary medicine prescription information for providing medicine to the user based on the medicine prescription information if a medicine-taking completion message is not received, and control the communicator to transmit the temporary medicine prescription information to the external apparatus.

2. The server of claim 1, wherein the external apparatus is an electronic apparatus which is installed in a medicine providing place and communicates with the server, and
wherein the medicine-taking completion message is received from a user terminal apparatus.

3. The server of claim 1, wherein the external apparatus is a user terminal apparatus which communicates with the server, and
wherein the medicine-taking completion message is received from the user terminal apparatus.

4. The server of claim 1, wherein the medicine prescription information comprises at least one of a type of prescribed medicine for the user, a number of times the user takes the prescribed medicine, and a time for taking the prescribed medicine.

5. The server of claim 4, wherein the temporary medicine prescription information is a medicine prescription which is used to prescribe a dose of medicine generated based on the medicine prescription information.

6. The server of claim 1, wherein the controller transmits the temporary medicine prescription information to the external apparatus and stores transmission event information in the storage unit if a request for a transmission of the temporary medicine prescription information is received from the external apparatus.

7. The server of claim 6, wherein the controller controls not to transmit the temporary medicine prescription information to the external apparatus if the request for the transmission of the temporary medicine prescription information is received after the transmission event information is stored.

8. The server of claim 1, wherein the external apparatus is a user terminal apparatus that communicates with the server,
wherein if a medicine-taking time in the medicine prescription information passes, the controller transmits a message to the user terminal apparatus to induce the user to take medicine and,
wherein if the medicine-taking completion message is not received at a preset time after the message is transmitted, the controller generates the temporary medicine prescription information.

9. A user terminal apparatus comprising:
a communicator configured to communicate with a server that stores medicine prescription information about a user; and
a controller configured to request the server to transmit temporary medicine prescription information, which is generated based on the medicine prescription information, to provide the user with prescribed medicine according to the medicine prescription information.

10. The user terminal apparatus of claim 9, wherein the controller controls the communicator to transmit a medicine-taking completion message to the server if the user takes the prescribed medicine according to the medicine prescription information,
wherein if the medicine-taking completion message is not received from the user terminal apparatus, the server generates the temporary medicine prescription information and transmits the temporary medicine prescription information to the user terminal apparatus.

11. A method of controlling a server, the method comprising:
generating temporary medicine prescription information for providing medicine to a user based on pre-stored medicine prescription information if a medicine-taking completion message is not received according to the pre-stored medicine prescription information; and
transmitting the temporary medicine prescription information to an external apparatus.

12. The method of claim 11, wherein the external apparatus is an electronic apparatus which is installed in a medicine providing place to communicate with the server, and
wherein the medicine-taking completion message is received from a user terminal apparatus.

13. The method of claim 11, wherein the external apparatus is a user terminal apparatus which communicates with the server, and
wherein the medicine-taking completion message is received from the user terminal apparatus.

14. A method of controlling a user terminal apparatus configured to communicate with a server that stores medicine prescription information about a user, the method comprising:
requesting the server to transmit temporary medicine prescription information generated based on the medicine prescription information to provide the user with prescribed medicine according to the medicine prescription information; and
receiving the temporary medicine prescription information from the server.

15. The method of claim 19, further comprising:
transmitting a medicine-taking completion message to the server if the user takes the prescribed medicine according to the medicine prescription information,
wherein the server generates the temporary medicine prescription information and transmits the temporary medicine prescription information to the user terminal apparatus if the medicine-taking completion message is not received from the user terminal apparatus.
